# EUROPÄISCHE PATENTSCHRIFT

(11) **EP 1 005 464 B1**
(45) Veröffentlichungstag und Bekanntmachung des Hinweises auf die Patenterteilung: **17.04.2002**
(21) Anmeldenummer: 99932699.4
(22) Anmeldetag: 18.06.1999
(51) Int. Cl.: C07D 311/78, C07D 311/94, C07D 311/96, C07D 493/04, G02B 5/23, C08K 5/15

(54) **PHOTOCHROME NAPHTHOPYRANE**
PHOTOCHROMIC NAPHTHOPYRANS
NAPHTOPYRANES PHOTOCHROMES

(30) Priorität: 19.06.1998 DE 19827411
(43) Veröffentlichungstag der Anmeldung: 07.06.2000
(73) Patentinhaber: Optische Werke G. Rodenstock, 80469 München (DE)
(72) Erfinder: MELZIG, Manfred, D-82234 Wessling (DE); MANN, Claudia, D-80634 München (DE); WEIGAND, Udo, D-80638 München (DE)
(74) Vertreter: Müller-Boré & Partner Patentanwälte
(86) Internationale Anmeldenummer: EP9904239
(87) Internationale Veröffentlichungsnummer: WO9967234

(56) Entgegenhaltungen:
- WO-A-96/14596
- US-A- 5 783 116

## Beschreibung

Die vorliegende Erfindung betrifft photochrome 2H-Naphtho[1,2-b]pyrane sowie deren Verwendung in Kunststoffen aller Art, insbesondere für ophthalmische Zwecke. Insbesondere betrifft die vorliegende Erfindung photochrome Naphthopyran-Verbindungen, bei denen an der f-Seite des Naphthopyrans ein weiteres Ringsystem gebunden ist.

Es sind verschiedene Farbstoffklassen bekannt, die bei Bestrahlung mit Licht bestimmter Wellenlängen, insbesondere Sonnenstrahlen, reversibel ihre Farbe wechseln. Dies rührt daher, daß diese Farbstoffmoleküle durch Energiezufuhr in Form von Licht in einen angeregten farbigen Zustand übergehen, den sie bei Unterbrechung der Energiezufuhr wieder verlassen, wodurch sie in ihren farblosen oder zumindest kaum gefärbten Normalzustand zurückkehren. Zu diesen photochromen Farbstoffen gehören beispielsweise die Naphthopyrane, die im Stand der Technik mit verschiedenen Substituenten bereits beschrieben wurden.

Pyrane, speziell Naphthopyrane und von diesen abgeleitete größere Ringsysteme, sind photochrome Verbindungen, die bis heute Gegenstand intensiver Untersuchungen sind. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen.

Eine geeignete Verbindungsklasse von Pyranen sind zum Beispiel die 2,2-Diaryl-2H-naphtho[1,2-b]pyrane. 2H-Naphtho[1,2-b]pyrane sind meist orange bis rote photochrome Verbindungen mit sehr langsamer Aufhellung nach Eindunkelung, wie beispielsweise die Vergleichsbeispiele 4 und 5 sowie das Vergleichsbeispiel a in US 5,066,818 zeigen.

Durch Substitution an den Kohlenstoffatomen in 5- und 6-Stellung des Naphthalinteils im Naphthopyran wurde versucht, hier Verbesserungen zu erzielen. Verbindungen dieser Art sind u.a. in US 5,458,814, US 5,573,712, US 5,650,098, US 5,656,206, US 5,658,500 und US 5,658,501 und WO 98/04937 beschrieben. Da weiterer Verbesserungsbedarf bestand, wurden in US 5,651,923 erstmals 2H-Naphtho[1,2-b]pyran-Systeme beschrieben, die an der f-Seite des Naphthopyrans, d.h. am Naphthalinteil, ein ankondensiertes Benzothieno-, Benzofurano- bzw. Indolosysteme aufweisen, wobei die Benzothieno-, Benzofurano- und Indolosysteme über den Heterozyklus an das Naphthalin gebunden sind. Nachteilig bei den drei beschriebenen Naphthofuro[2',3':3,4]-und Naphthofuro[3',2':3,4]-naphtho[1,2-b]pyranen der Beispiele 4, 5 und 6 in US 5,651,923 ist, daß diese eine sehr langsame Aufhellung und/oder sehr geringe photochrome Leistung (ΔOD) besitzen. Auch Indolonaphthopyrane zeigen gleichermaßen eine extrem langsame Aufhellung. In WO 99/20619 werdenPentahydrophenanthro[9,10-b]pyraneundTetrahydrocyclopenta[c]-naphtho[1,2-b]pyrane beschrieben, die ebenfalls den Nachteil aufweisen, daß sie langsame und somit für eine praktische Verwendung in Sonnenschutzgläsern unzufriedenstellende Aufhellungsgeschwindigkeit zeigen.

Außerdem ist bei den beschriebenen Systemen ein aufwendiger und langer Syntheseweg nötig. Ferner ist durch die Konjugationsmöglichkeit mit einem weiteren aromatischen Ring(system) eine bathochrome Verschiebung gegenüber der Grundabsorption des Naphthopyransystems zu beobachten, die oft nicht gewünscht wird. Dies gilt in gleicher Weise für die Verbindungen in US 5,645,767, die ausschließlich Indeno[2,1-f]naphtho[1,2-b]pyrane betrifft. Weitere Fortführungen sind in US 5,698,141 und US 5,723,072 zu finden, die noch zusätzlich ankondensierte un-, mono- oder disubstituierte Heterozyklen aufweisen.

Die bekannten Verbindungen sind jedoch zudem mit Nachteilen verbunden, die bei Verwendung dieser photochromen Farbstoffe in Sonnenschutzgläsern den Tragekomfort des Brillenträgers wesentlich beeinträchtigen. Die bekannten Farbstoffe weisen eine nicht ausreichend langwellige Absorption im angeregten wie im nicht angeregten Zustand auf. Dies führt auch bei Kombinationen mit weiteren photochromen Farbstoffen zu Problemen. Es liegt eine zu hohe Temperaturempfindlichkeit bezüglich der Eindunkelung vor, wobei gleichzeitig häufig eine zu langsame Aufhellung eintritt. Darüberhinaus besitzen die beschriebenen Farbstoffe eine ungenügende Lebensdauer und damit unzureichende Haltbarkeit der Sonnenschutzgläser. Letzteres macht sich in schnell nachlassender Leistung und/oder starker Vergilbung bemerkbar. Zudem ist die Synthese der im Stand der Technik beschriebenen Verbindungen in der Regel aufwendig und langwierig und gestattet es meist nicht, weitere optisch interessante Eigenschaften, wie Nichtlinearität, in die jeweiligen Strukturen einzubauen.

Somit liegt der vorliegenden Erfindung die Aufgabe zugrunde, neue photochrome Verbindungen bereitzustellen, die verbesserte Eigenschaften gegenüber den im Stand der Technik beschriebenen Strukturen besitzen sollen. Die photochromen Verbindungen sollen sich insbesondere dadurch auszeichnen, daß sie in der lichtangeregten Form gegenüber vergleichbaren Verbindungen aus dem Stand der Technik eine schnellere Kinetik und ein besseres Verhalten im Lebensdauertest aufweisen.

Diese Aufgabe wird durch die in den Ansprüchen gekennzeichneten Gegenstände gelöst. Insbesondere werden photochrome 2H-Naphtho-[1,2-b]pyrane mit der allgemeinen Formel (I) bereitgestellt: 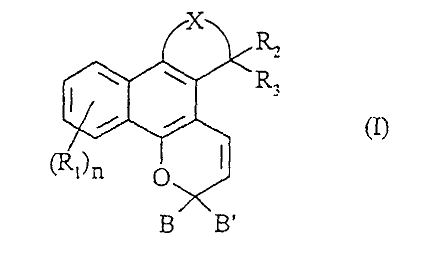 worin
- X: ein Ringglied mit 2 bis 4, gesättigten und/oder ungesättigten Kohlenstoffatomen ist, von denen maximal eines durch ein Heteroatom, ausgewählt aus der Gruppe, bestehend aus O, S und NR₄, ersetzt sein kann, wobei R₄ einen geradkettigen oder verzweigtkettigen (C₁-C₆)-Alkylrest, einen substituierten oder unsubstituierten (C₅-C₇)-Cycloalkylrest, einen substituierten oder unsubstituierten Phenylrest oder einen substituierten oder unsubstituierten Benzylrest darstellt;
- R₁: ein Substituent ist, ausgewählt aus der Gruppe A, bestehend aus einem (C₁-C₆)-Alkylrest, einem (C₁-C₆)-Alkoxyrest, einem (C₃-C₇)-Cycloalkylrest, der ein oder mehrere Heteroatome aufweisen kann, einem Phenylrest, einer Hydroxygruppe, Brom, Chlor und Fluor, wobei n 0, 1 oder 2 ist;
- R₂, R₃: unabhängig voneinander Reste sind, ausgewählt aus der Gruppe G, bestehend aus Wasserstoff, Hydroxy, einem (C₁-C₆)-Alkylrest, einem (C₁-C₆)-Alkoxyrest, einem (C₃-C₇)-Cycloalkylrest, einem un-, mono- und disubstituierten Phenylrest und einem un-, mono- und disubstituierten Naphthylrest und den aromatischen Resten, ausgewählt aus der Gruppe C, bestehend aus Benzol, Naphthalin, Phenanthren, Pyridin, Chinolin, Furan, Thiophen, Pyrrol, Benzofuran, Benzothiophen, Indol und Carbazol, wobei der oder die Substituenten der vorgenannten aromatischen Reste aus der Gruppe A ausgewählt ist bzw. sind;
oder R₂ und R₃ zusammen unter Einrechnung des Spiro-Kohlenstoffatoms einen 5 bis 11-gliedrigen Ring bilden, an welchen ein oder mehrere aromatische oder heteroaromatische Ringsysteme annelliert sein kann bzw. können, wobei das bzw. die aromatische(n) oder heteroaromatische(n) Ringsystem(e) eines aus der vorgenannten Gruppe C ist bzw. sind;
oder R₂ und R₃ unter Bildung einer Carbonylgruppe zusammen für ein Sauerstoffatom stehen;
mit der Maßgabe, daß, wenn X -(CH₂)₂- oder -(CH₂)₃- ist, R₂ und R₃ nicht gleichzeitig Wasserstoff sind;
- B, B': unabhängig voneinander aus einer der folgenden Gruppen a), b) oder c) ausgewählt sind, wobei
a) mono-, di- und trisubstituierte Arylreste sind, wobei der Arylrest Phenyl oder Naphthyl ist;
b) unsubstituierte, mono- und disubstituierte Heteroarylreste sind, wobei der Heteroarylrest Pyridyl, Furanyl, Benzofuran-2-yl, Benzofuran-3-yl, Thienyl, Benzothien-2-yl oder Benzothien-3-yl ist; wobei die Substituenten der Aryl- oder Heteroarylreste in a) und b) solche sind, ausgewählt aus einer Gruppe, bestehend aus Hydroxy, Amino, Mono-(C₁-C₆)-alkylamino, Di-(C₁-C₆)-alkylamino, am Aromaten un-, mono- oder disubstituiertem Mono- und Diphenylamino, Piperidinyl, Morpholinyl, Carbazolyl, un-, mono- und disubstituiertem Pyrryl, (C₁-C₆)-Alkyl, (C₁-C₆)-Alkoxy, Brom, Chlor und Fluor, wobei die vorgenannten aromatischen und heteroaromatischen Ringsysteme mit (C₁-C₆)-Alkyl, (C₁-C₆)-Alkoxy, Brom, Chlor und Fluor substituiert sein können;
c) Struktureinheiten mit den folgenden Formeln (V) und (W) sind 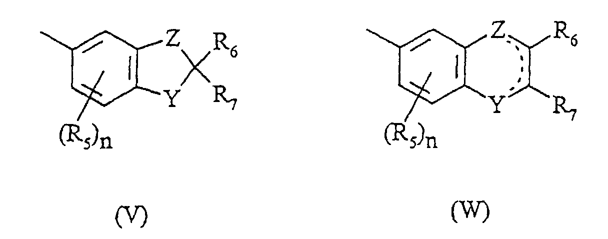 worin
   Y und Z unabhängig voneinander O, S, CH, CH₂ oder NR₈ sind, der Rest R₈ ausgewählt ist aus der Gruppe D, bestehend aus (C₁-C₆)-Alkyl, (C₁-C₆)-Acyl und Wasserstoff, und R₅ jeweils ein Substituent aus der Gruppe A ist, wobei n wie oben definiert ist; und R₆ und R₇ unabhängig voneinander Wasserstoff und/oder einen (C₁-C₆)-Alkylrest darstellen, mit der Maßgabe, daß, wenn Y in der Formel (V) NR₈ ist, Z Kohlenstoff ist,
   oder
d) B und B' miteinander einen un-, mono- oder disubstituierten Fluoren-9-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Fluorenosubstituenten aus der Gruppe A ausgewählt sind.
Gemäß der vorliegenden Erfindung werden durch einfache Ringbildung an der f-Seite des Naphthopyrangerüsts Verbindungen bereitgestellt, deren Absorptionswellenlängen gegenüber dem Grundgerüst kaum verschoben sind, aber gleichzeitig in überraschender Weise gegenüber entsprechenden Verbindungen aus dem Stand der Technik eine wesentlich verbesserte Aufhellung zeigen. Gegenüber den in WO 99/20619 beschriebenen Pentahydrophenanthro[9,10-b]pyranen und Tetrahydrocyclopenta[c]-naphtho[1,2-b]pyranen, die sich von den erfindungsgemäßen Naphthopyranen dadurch unterscheiden, daß an der f-Seite des Naphthopyrangerüsts ein unsubstituierter Cyclopentan- bzw. unsubstituierter Cyclohexanring gebunden ist, d.h. X -(CH₂)₂- bzw. -(CH₂)₃- ist und R₂ und R₃ jeweils Wasserstoff sind, zeigen die erfindungsgemäßen Verbindungen deutlich schnellere Aufhellung und wesentliche Verbesserungen in der Lebensdauer. Bei den erfindungsgemäßen Verbindungen tritt darüberhinaus keine Vergilbung mehr auf.

In einer bevorzugten Ausführungsform sind die Kohlenstoffatome in dem Ringglied X gesättigt, wodurch an der f-Seite des Naphthopyrans entsprechende, mit den Resten R₂ und R₃ substituierte cycloaliphatische Ringsysteme gebildet werden.

Die Kohlenstoffatome im Ringglied X können nicht nur unsubstituiert, sondern auch mono- oder disubstituiert sein, wobei der oder die Substituenten dann aus der Gruppe A, bestehend aus (C₁-C₆)-Alkyl, (C₁-C₆)-Alkoxy, Phenyl, Brom, Chlor und Fluor, ausgewählt sein können.

Gemäß der vorliegenden Erfindung werden auch photochrome 2H-Naphtho-[1,2-b]pyrane mit der Formel (I) bereitgestellt, die strukturell an der f-Seite des Naphthopyransystems annellierte Spirosysteme aufweisen, wobei das Spirosystem durch das über das Ringglied X an der f-Seite des Naphthopyrans gebildete Ringsystem und den am zentralen Spiro-Kohlenstoffatom gebundenen Resten R₂ und R₃ gebildet wird. Die Reste R₂ und R₃ bilden dabei zusammen unter Einrechnung bzw. Einbindung des Spiro-Kohlenstoffatoms einen 5- bis 11-gliedrigen Ring, vorzugsweise einen 5- bis 7-gliedrigen Ring, an welchen wiederum aromatische oder heteroaromatische Ringsysteme annelliert sein können. Mit anderen Worten können benachbarte Kohlenstoffe in dem von R₂ und R₃ gebildeten Teil eines Spirosystems zu einem weiteren Ringsystem gehören, nämlich einem Ringsystem aus der Gruppe C, bestehend aus Benzol, Naphthalin, Phenanthren, Pyridin, Chinolin, Furan, Thiophen, Pyrrol, Benzofuran, Benzothiophen, Indol und Carbazol. Selbstverständlich können diese Ringsysteme der Gruppe C sowohl un- als auch mono- oder disubstituiert sein, wobei der oder die Substituenten aus der oben definierten Gruppe A ausgewählt sind.

Erfindungsgemäßbevorzugte photochrome2H-Naphtho[1,2-b]pyrane weisen die nachfolgende allgemeine Formeln (II) auf: 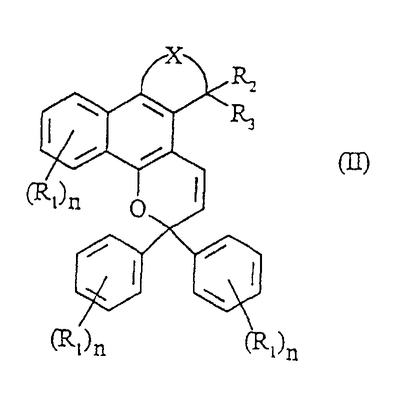 worin n, R₁, R₂, R₃, und X wie vorgenannt definiert sind, mit der Maßgabe, daß die Reste R₁ und n jeweils gleich oder verschieden sein können.

In einer weiteren Ausführungsform werden photochrome 2H-Naphtho-[1,2-b]pyrane bereitgestellt, welche die nachfolgende allgemeine Formel (III) aufweisen: 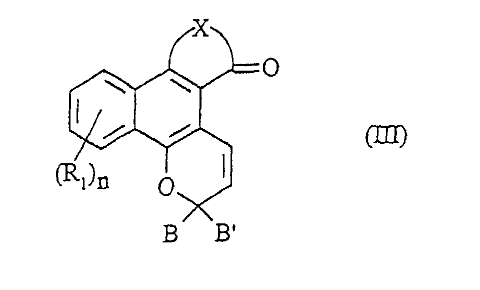 worin n, R₁, X, B und B' wie vorgenannt definiert sind.

Besonders bevorzugte Verbindungen gemäß der vorliegenden Erfindung sind:
1) Spiro-9'-xanthen-5-[2-(4-methoxyphenyl)-2-phenyl-5,6,7,8-tetrahydro-6,6,7,7-tetramethyl-phenanthro[9,10-b]pyran]
2) Spiro-9'-fluoren-5-{2-[4-(N-morpholinyl)phenyl]-2-phenyl-5,6,7,8-tetrahydro-phenanthro[9,10-b]pyran}
3) 5-Hydroxy-2,2,5-triphenyl-5,6,7,8-tetrahydro-phenanthro[9,10-b]pyran
4) Spiro-9'-{(9,10-dihydroanthracen)-5-[2,2-bis(4-methoxyphenyl)]-cyclopenta[f]naphtho[1,2-b]pyran}
5) 2-[4-(N-Morpholinyl)phenyl]-2-phenyl-5-oxo-cyclopenta[f]naphthopyran
6) 2-[4-(N-Morpholinyl)phenyl]-2-phenyl-5,6,7,8-tetrahydro-5-oxo-phenanthro[9,10-b]pyran
7) Spiro-9'-fluoren-5-[2-(4-methoxyphenyl)-2-phenyl-cyclopenta-[f]naphthro[1,2-b]pyran]
8) Spiro-9'-xanthen-5-{2-[4-(N-morpholinyl)phenyl]-2-phenyl-5,6,7,8-tetrahydro-6,6,7,7-tetramethyl-phenanthro[9,10-b]pyran}
9) Spiro-9'-fluoren-5-[2-(4-methoxyphenyl)-2-phenyl-cyclohepta[f]naphtho-[1,2-b]pyran] und
10) Spiro-9'-fluoren-5-[2-(4-methoxyphenyl)-2-phenyl-oxepano[3,2-f]naphtho[1,2-b]pyran].

Die erfindungsgemäßen photochromen 2H-Naphtho[1,2-b]pyrane mit der allgemeinen Formel (I) lassen sich nach den auf dem Fachgebiet grundsätzlich bekannten Syntheseprinzipien herstellen. In diesem Zusammenhang wird insbesondere auf die in WO 99/15518 und der deutschen Patentanmeldung 199 02 771.4 beschriebenen Synthesewege verwiesen. Beispielsweise können die erfindungsgemäßen photochromen 2H-Naphtho[1,2-b]pyrane nach dem folgenden allgemeinen Umsetzungsschema, ohne darauf beschränkt zu sein, hergestellt werden, wobei n, X, R₁, R₂, R₃, B und B' wie vorgenannt definiert sind.

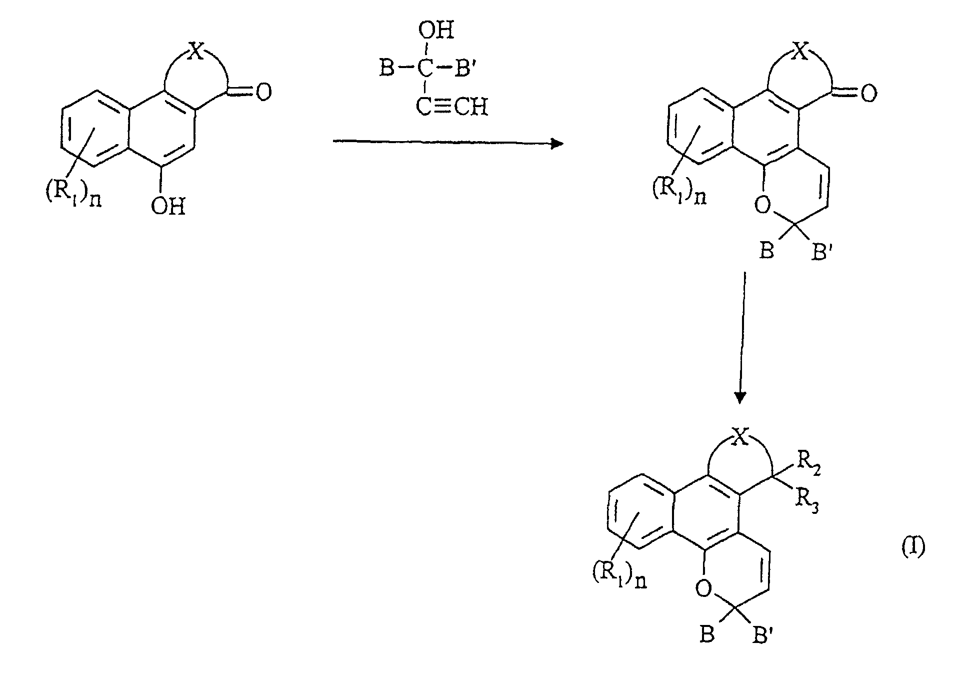

Die Derivatisierung der Ketogruppe im zweiten Schritt dieses allgemeinen Umsetzungsschemas kann dabei nach den üblichen bekannten Verfahren, wie z.B. Umsetzung mit entsprechenden Grignardreagenzien, etc., erfolgen.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die photochromen Naphthopyran-Farbstoffe gemäß der vorliegenden Erfindung in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen Naphthopyrane beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Naphthopyran-Farbstoffe durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfaßt beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen 2H-Naphtho-[1,2-b]pyrane beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

Nachfolgend wird die Herstellung beispielhaft ausgewählter, erfindungsgemäßer 2H-Naphtho[1,2-b]pyrane detailliert erläutert, wobei diese Beispiele selbstverständlich den Schutzbereich der vorliegenden Erfindung nicht beschränken, sondern nur zur Veranschaulichung dienen sollen.

### Beispiele

### Beispiel 1: Herstellung von Spiro-9'-xanthen-5-[2-(4-methoxyphenyl)-2-phenyl-5,6,7,8-tetrahydro-6,6,7,7-tetramethylphenanthro[9,10-b]pyran]

i) 24,5 g Aluminiumchlorid und 14,5 g Tetramethylbernsteinsäureanhydrid (A. v. Auwers, N. Ungemach, Chem. Ber. 1935, 68, 349) wurden unter Rühren und Kühlen mit Eiswasser mit 14,7 g Methoxynaphthalin portionsweise versetzt. Nach Auftauen und Rühren der Mischung über Nacht wurde der Ansatz auf Eiswasser gegossen. Anschließend wurde der entstandene Niederschlag abgesaugt. Die Säure wurde über ihr Natriumsalz gereinigt und aus Essigsäure umkristallisiert. Der farblose Feststoff (18 g) wurde mittels NMR-Spektroskopie als β-(4-Methoxy-1-naphthoyl)-α,α,β,β-tetramethylpropionsäure identifiziert.
ii) 10 g der derart erhaltenen Ketosäure wurden mit 10 g Kaliumhydroxid und 8 ml 80%igem Hydrazinhydrat in 50 ml Diethylenglykol 8 h auf 130 bis 135 °C erhitzt. Nach Abdestillieren des Wassers und des überschüssigen Hydrazinhydrats wurde 3 h auf etwa 210 °C erhitzt, anschließend auf 90 °C abgekühlt und auf eine Mischung aus 200 g Eis und 50 ml konzentrierter Salzsäure gegossen. Der entstandene Niederschlag wurde abgesaugt und neutral gewaschen. Der Feststoff wurde anschließend mit 14 ml Dimethylsulfat in 80 ml 10%iger Natronlauge bei 40 bis 50 °C gerührt. Der intermediär gebildete Ester wurde anschließend durch Erhitzen unter Rückfluß zum Sieden verseift. Nach Abkühlen der Lösung wurde angesäuert, der entstandene Niederschlag abgesaugt und neutral gewaschen. Das Produkt (7,5 g) wurde mittels NMR-Spektroskopie als γ-(4-Methoxy-1-naphthyl)-α,α,β,β-tetramethylbuttersäure identifiziert.
iii) Eine Suspension aus 5 g der in Schritt ii) erhaltenen Säure in 100 ml wasserfreiem Ether und 2 Tropfen Pyridin wurde mit 4 ml Thionylchlorid versetzt und 1 h unter Rückfluß zum Sieden erhitzt. Nach Abkühlen wurde das Lösungsmittel im Vakuum abdestilliert und der Rückstand in 50 ml gelöst und unter Rühren und Kühlen mit Eiswasser mit 8 ml Zinntetrachlorid versetzt. Die Mischung wurde 15 min gerührt und anschließend auf eine Mischung aus 100 ml Eiswasser und 25 ml konzentrierter Salzsäure gegossen. Die organische Phase wurde abgetrennt und je einmal mit verdünnter Salzsäure, Wasser und Ammoniumhydroxid-Lösung gewaschen. Nach Abdestillieren des Lösungsmittels im Vakuum blieb ein hellgelber Rückstand (4,1 g) zurück, der mittels NMR-Spektroskopie als 1,2,3,4-Tetrahydro-9-methoxy-2,2,3,3-tetramethyl-1-oxo-phenanthren identifiziertwurde.
iv) Das in Schritt iii) erhaltene Reaktionsprodukt (4 g) wurde in einer Mischung aus 20 ml 48%iger Bromwasserstoffsäure und 20 ml Eisessig 3 h unter Rückfluß zum Sieden erhitzt. Nach Abkühlen wurde der Ansatz auf Wasser gegossen, alkalisch gemacht und zweimal mit Ether gewaschen. Die Lösung wurde angesäuert und der entstandene Niederschlag abgesaugt und neutral gewaschen. Das Produkt (3,5 g) wurde mittels NMR-Spektroskopie als 1,2,3,4-Tetrahydro-9-hydroxy-2,2,3,3-tetramethyl-1-oxo-phenanthren identitiziert.
v) 3 g des in Schritt iv) erhaltenen Phenanthrenderivats wurden mit 4,6 g 1-(4-Methoxyphenyl)-1-phenyl-1-propinol (hergestellt aus 4-Methoxybenzophenon und Natriumacetylid in DMSO) in 100 ml Toluol suspendiert. Nach Zugabe einer Spatelspitze 4-Toluolsulfonsäure wurde die Mischung 2 h unter Rückfluß zum Sieden erhitzt, wobei Lösung eintrat. Nach Abkühlen wurde die Hälfte des Lösungsmittels im Vakuum abdestilliert und die restliche Lösung an Aluminiumoxid (Wassergehalt 3%) mit einer Dichlormethan-Hexan-Mischung (1/1) chromatographiert. Zur endgültigen Reinigung wurde das Rohprodukt in 50 ml Methanol digeriert. Der Feststoff wurde abgesaugt, mit Methanol gewaschen und getrocknet. Das hellgelbe Pulver (4,1 g) wurde mittels NMR-Spektroskopie als 2-(4-Methoxyphenyl)-2-phenyl-5,6,7,8-tetrahydro-6,6,7,7-tetramethyl-5-oxo-phenanthro[9,10-b]pyran (4 g) identifiziert.
vi) 2 g des in Schritt v) erhaltenen Reaktionsproduktes wurden in 50 ml wasserfreiem THF unter Rühren gelöst und mit 2 Äquivalenten 2-Phenoxyphenylmagnesiumbromid (hergestellt aus 2-Bromdiphenylether und Magnesiumspänen in THF-Lösung) versetzt. Das Gemisch wurde 10 h bei Raumtemperatur gerührt und mit wäßriger Ammoniumchlorid-Lösung hydrolysiert. Die organische Phase wurde nach Zugabe von 100 ml Dichlormethan abgetrennt, mit Wasser gewaschen und über Natriumsulfat getrocknet. Nach Abdestillieren des Lösungsmittels wurde der Rückstand mit Methanol digeriert. Mittels NMR-Spektroskopie wurde der Rückstand als 5-Hydroxy-2-(4-methoxyphenyl)-5-(2-phenoxyphenyl)-2-phenyl-5,6,7,8-tetrahydro-6,6,7,7-tetramethylphenanthro[9,10-b]pyran (1,1 g) identifiziert, das als Rohprodukt weiter eingesetzt wurde.
vii) 0,8 g des in Schritt vi) erhaltenen Reaktionsproduktes wurden nach R. G. Clarkson, M. Gomberg, J. Am. Chem. Soc. 1930, Seite 2881, in 30 ml Eisessig in der Hitze cyclisiert. Nach Zugabe eines Tropfens Salzsäure wurde noch 5 min zum Sieden erhitzt und anschließend so viel Wasser zugegeben, bis die Reaktionslösung trüb wurde. Nach dem Abkühlen wurde der ausgefallene Niederschlag abgesaugt, neutral gewaschen und sorgfältig getrocknet. Zur endgültigen Reinigung wurde der Feststoff in 30 ml Dichlormethan gelöst und an Aluminiumoxid (Wassergehalt 3%) mit einer Dichlormethan-Hexan-Mischung (1/1) chromatographiert. Nach Digerieren mit Hexan wurde ein beigefarbenes Pulver (0,3 g) erhalten, das mittels NMR-Spektroskopie als Spiro-9'-xanthen-5-[2-(4-methoxyphenyl)-2-phenyl-5,6,7,8-tetrahydro-6,6,7,7-tetramethylphenanthro[9,10-b]pyran] identifiziert wurde.

### BEISPIEL 2: Herstellung von Spiro-9'-fluoren-5-{2-[4-(N-morpholinyl)phenyl]-2-phenyl-5,6,7,8-tetrahydrophenanthro[9,10-b]pyran}

Ausgehend von Methoxynaphthalin und Bernsteinsäureanhydrid wurde zunächst analog den Schritten i) bis iii) von Beispiel 1 (vgl. W. E. Bachmann und D. W. Holmes (J. Chem. Soc. 1940, 62, Seite 2750) 1,2,3,4-Tetrahydro-9-methoxy-1-oxophenanthren hergestellt. Die Demethylierung (Schritt iv) erfolgte gemäß G. A. R. Kon und F. C. J. Ruzicka (J. Chem. Soc. 1936, Seite 187).

Die anschließende Reaktionsführung erfolgte analog Beispiel 1, mit der Ausnahme, daß im Schritt v) die Umsetzung mit 1-[4-(N-Morpholinyl)phenyl]-1-phenyl-1-propinol [hergestellt aus 4-(N-Morpholinyl)benzophenon (H. Kotsuki, Synthesis 1990, Seite 1145) und Natriumacetylid in DMSO] anstelle von 1-(4-Methoxyphenyl)-1-phenyl-1-propinol durchgeführt wurde. Es wurde 2-[4-(N-Morpholinyl)phenyl]-2-phenyl-5,6,7,8-tetrahydro-5-oxo-phenanthro[9,10-b]pyran erhalten, das analog Beispiel 1 im nachfolgenden Schritt vi) mit 2-Biphenylylmagnesiumbromid (hergestellt aus 2-Brombiphenyl und Magnesiumspänen in THF-Lösung) zu 5-Hydroxy-5-(2-biphenylyl)-2-[4-(N-morpholinyl)phenyl]-2-phenyl-5,6,7,8-tetrahydrophenanthro(9,10-b]pyran] umgesetzt wurde. Analog Beispiel 1 erfolgte im anschließenden Schritt vii) die Cyclisierung zu Spiro-9'fluoren-5-{2-[4-(N-morpholinyl)phenyl]-2-phenyl-5,6,7,8-tetrahydrophenanthro[9,10-b]pyran} (0,3 g), wie durch NMR-Spektroskopie bestätigt wurde.

### BEISPIEL 3: Herstellung von 5-Hydroxy-2,2,5-triphenyl-5,6,7,8-tetrahydrophenanthro[9,10-b]pyran

Die Herstellungsweise erfolgte analog Beispiel 2, mit der Ausnahme, daß im Schritt v) die Umsetzung mit 1,1-Diphenyl-1-propinol [hergestellt aus Benzophenon und Natriumacetylid in DMSO] anstelle von 1-[4-(N-Morpholinyl)phenyl]-1-phenyl-1-propinol durchgeführt wurde. Es wurde 2,2-Diphenyl-5,6,7,8-tetrahydro-5-oxo-phenanthro[9,10-b]pyran erhalten, welches analog Beispiel 1 in Schritt vi) mit 2 Äquivalenten einer 2 molaren Lösung von Phenylmagnesiumchlorid in THF umgesetzt wurde. Das derart erhaltene Rohprodukt wurde an Aluminiumoxid (Wassergehalt 3%) mit Dichlormethan chromatographiert. Das beigefarbene Produkt (0,5 g) wurde mittels NMR-Spektroskopie als 5-Hydroxy-2,2,5-triphenyl-5,6,7,8-tetrahydrophenanthro[9,10-b]pyran identifiziert.

### BEISPIEL 4: Herstellung von Spiro-9'-{(9,10-dihydroanthracen)-5-[2,2-bis(4-methoxyphenyl)]-cyclopenta[c]naphtho[1,2-b]pyran}

Anstelle von 1,2,3,4-Tetrahydro-9-hydroxy-2,2,3,3-tetramethyl-1-oxo-phenanthren wurde in Schritt v) gemäß Beispiel 1 als Ausgangsmaterial 4,5-Benzo-8-hydroxy-indan-1-on (vgl. T. Sasaki, K. Kanematsu, K. Hayakawa, A. Kondo, J. Org. Chem. 1973, 38, Seite 4100) eingesetzt. Dieses wurde mit 1,1-Bis(4-methoxyphenyl)-1-propinol (hergestellt aus 4,4'-Dimethoxybenzophenon und Natriumacetylid in DMSO) umgesetzt. Es wurde 2,2-Bis(4-methoxyphenyl)-5-oxo-cyclopenta[c]naphthopyran erhalten, welches analog Beispiel 1 im nachfolgenden Schritt vi) mit 2-Benzylphenylmagnesiumbromid (hergestellt aus 2-Bromdiphenylmethan und Magnesium in THF-Lösung) zu 5-(2-Benzylphenyl)-5-hydroxy-2,2-bis(4-methoxyphenyl)-cyclopenta[f]naphthopyran umgesetzt wurde. Analog Beispiel 1 erfolgte im anschließenden Schritt vii) die Cyclisierung zu Spiro-9'-{(9,10-dihydroanthracen)-5-[2,2-bis(4-methoxyphenyl)]-cyclopenta[c]-naphtho[1,2-b]pyran} (0,4 g), wie mittels NMR-Spektroskopie bestätigt wurde.

## Patentansprüche

1. Photochrome 2H-Naphtho[1,2-b]pyrane mit der allgemeinen Formel (I): 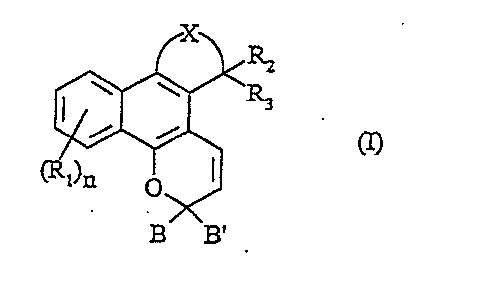 worin
X ein Ringglied mit 2 bis 4, gesättigten und/oder ungesättigten Kohlenstoffatomen ist, von denen maximal eines durch ein Heteroatom, ausgewählt aus der Gruppe, bestehend aus O, S und NR₄, ersetzt sein kann, wobei R₄ einen geradkettigen oder verzweigtkettigen (C₁-C₆)-Alkylrest, einen substituierten oder unsubstituierten (C₅-C₇)-Cycloalkylrest, einen substituierten oder unsubstituierten Phenylrest oder einen substituierten oder unsubstituierten Benzylrest darstellt, und wobei die Kohlenstoffatome im Ringglied X unsubstituiert, mono- oder disubstituiert sein können, wobei der oder die Substituenten dann aus der Gruppe A, bestehend aus (C₁-C₆)-Alkyl, (C₁-C₆)-Alkoxy, Phenyl, Brom, Chlor und Fluor, ausgewählt sein können;
R₁ ein Substituent ist, ausgewählt aus der Gruppe A, bestehend aus einem (C₁-C₆)-Alkylrest, einem (C₁-C₆)-Alkoxyrest, einem (C₃-C₇)-Cycloalkylrest, der ein oder mehrere Heteroatome aufweisen kann, einem Phenylrest, einer Hydroxygruppe, Brom, Chlor und Fluor, wobei n 0, 1 oder 2 ist;
R₂, R₃ unabhängig voneinander Reste sind, ausgewählt aus der Gruppe G, bestehend aus Wasserstoff, Hydroxy, einem (C₁-C₆)-Alkylrest, einem (C₁-C₆)-Alkoxyrest, einem (C₃-C₇)-Cycloalkylrest, einem un-, mono- und disubstituierten Phenylrest und einem un-, mono- und disubstituierten Naphthylrest und den aromatischen Resten, ausgewählt aus der Gruppe C, bestehend aus Benzol, Naphthalin, Phenanthren, Pyridin, Chinolin, Furan, Thiophen, Pyrrol, Benzofuran, Benzothiophen, Indol und Carbazol, wobei der oder die Substituenten der vorgenannten aromatischen Reste aus der Gruppe A ausgewählt ist bzw. sind;
oder R₂ und R₃ zusammen unter Einrechnung des Spiro-Kohlenstoffatoms einen 5 bis 11-gliedrigen Ring bilden, an welchen ein oder mehrere aromatische oder heteroaromatische Ringsysteme annelliert sein kann bzw. können, wobei das bzw. die aromatische(n) oder heteroaromatische(n) Ringsystem(e) eines aus der vorgenannten Gruppe C ist bzw. sind;
oder R₂ und R₃ unter Bildung einer Carbonylgruppe zusammen für ein Sauerstoffatom stehen;
mit der Maßgabe, daß, wenn X -(CH₂)₂- oder -(CH₂)₃- ist, R₂ und R₃ nicht gleichzeitig Wasserstoff sind;
B, B' unabhängig voneinander aus einer der folgenden Gruppen a), b) oder c) ausgewählt sind, wobei
a) mono-, di- und trisubstituierte Arylreste sind, wobei der Arylrest Phenyl oder Naphthyl ist;
b) unsubstituierte, mono- und disubstituierte Heteroarylreste sind, wobei der Heteroarylrest Pyridyl, Furanyl, Benzofuran-2---yl, Benzofuran-3-yl, Thienyl, Benzothien-2-yl oder Benzothien-3-yl ist;
wobei die Substituenten der Aryl- oder Heteroarylreste in a) und b) solche sind, ausgewählt aus einer Gruppe, bestehend aus Hydroxy, Amino, Mono-(C₁-C₆)-alkylamino, Di-(C₁-C₆)-alkylamino, am Aromaten un-, mono- oder disubstituiertem Mono- und Diphenylamino, Piperidinyl, Morpholinyl, Carbazolyl, un-, mono- und disubstituiertem Pyrryl, (C₁-C₆)-Alkyl, (C₁-C₆)-Alkoxy, Brom, Chlor und Fluor, wobei die vorgenannten aromatischen und heteroaromatischen Ringsysteme mit (C₁-C₆)-Alkyl, (C₁-C₆)-Alkoxy, Brom, Chlor und Fluor substituiert sein können;
c) Struktureinheiten mit den folgenden Formeln (V) und (W) sind 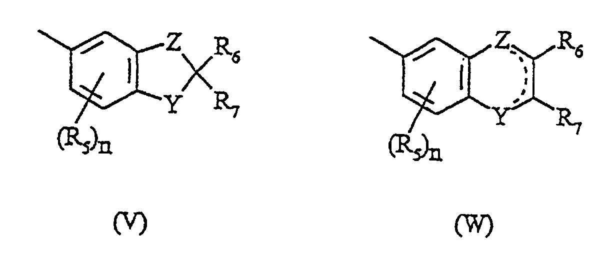 worin
Y und Z unabhängig voneinander O, S, CH, CH₂ oder NR₈ sind, der Rest R₈ ausgewählt ist aus der Gruppe D, bestehend aus (C₁-C₆)-Alkyl, (C₁-C₆)-Acyl und Wasserstoff, und R₅ jeweils ein Substituent aus der Gruppe A ist, wobei n wie oben definiert ist; und R₆ und R₇ unabhängig voneinander Wasserstoff und/oder einen (C₁-C₆)-Alkylrest darstellen, mit der Maßgabe, daß, wenn Y in der Formel (V) NR₈ ist, Z Kohlenstoff ist,
oder
d) B und B' miteinander einen un-, mono- oder disubstituierten Fluoren-9-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Fluorenosubstituenten aus der Gruppe A ausgewählt sind.

2. Photochrome 2H-Naphtho[1,2-b]pyrane nach Anspruch 1, wobei die Kohlenstoffatome in dem Ringglied X gesättigt sind.

3. Photochrome 2H-Naphtho(1/2-b]pyrane nach Anspruch 1 oder 2, wobei die Kohlenstoffatome im Ringglied X mono- oder disubstituiert sind, wobei der oder die Substituenten aus der Gruppe A, bestehend aus (C₁-C₆)-Alkyl, (C₁-C₆)-Alkoxy, Phenyl, Brom, Chlor und Fluor, ausgewählt sind.

4. Photochrome 2H-Naphtho[1,2-b]pyrane nach einem der vorhergehenden Ansprüche, welche die allgemeine Formel (II) aufweisen: 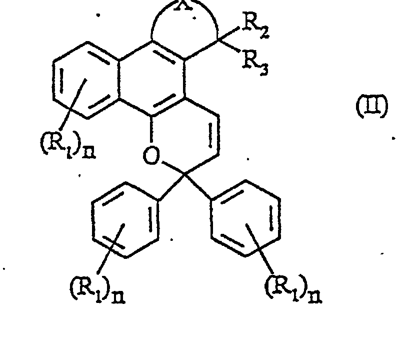 worin n, R₁, R₂, R₃ und X wie vorgenannt definiert sind, mit der Maßgabe, daß die Reste R₁ und n jeweils gleich oder verschieden sein können.

5. Photochrome 2H-Naphtho[1,2-b]pyrane nach einem der vorhergehenden Ansprüche, welche die allgemeine Formel (III) aufweisen: 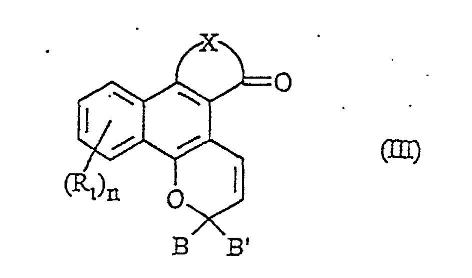 worin n, R₁, X, B und B' wie vorgenannt definiert sind.

6. Photochrome 2H-Naphtho[1,2-b]pyrane nach einem der vorhergehenden Ansprüche, welche
Spiro-9'-xanthen-5-[2-(4-methoxyphenyl)-2-phenyl-5,6,7,8-tetrahydro-6,6,7,7-tetramethyl-phenanthro[9,10-b]pyran],
Spiro-9'-fluoren-5-{2-[4-(N-morpholinyl)phenyl]-2-phenyl-5,6,7,8-tetrahydrophenanthro[9,10-b]pyran),
5-Hydroxy-2,2,5-triphenyl-5,6,7,8-tetrahydro-phenanthro[9,10-b]pyran,
Spiro-9'-{(9,10-dihydroanthracen)-5-[2,2-bis(4-methoxyphenyl)]-cyclopenta[f]naphtho[1,2-b]pyran},
2-[4-(N-Morpholinyl)phenyl]-2-phenyl-5-oxo-cyclopenta[f]naphthopyran,
2-[4-(N-Morpholinyl)phenyl]-2-phenyl-5,6,7,8-tetrahydro-5-oxo-phenanthro[9,10-b]pyran,
Spiro-9'-fluoren-5-[2-(4-methoxyphenyl)-2-phenyl-cyclopenta-[f]naphtho-[1,2-b]pyran],
Spiro-9'-xanthen-5-{2-[4-(N-morpholinyl)phenyl]-2-phenyl-5,6,7,8-tetrahydro-6,6,7,7-tetramethyl-phenanthro[9,10-b]pyran),
Spiro-9'-fluoren-5-[2-(4-methoxyphenyl)-2-phenyl-cyclohepta[f]naphtho-[1,2-b]pyran] und
Spiro-9'-fluoren-5-[2-(4-methoxyphenyl)-2-phenyl-oxepano[3,2-f]naphtho[1,2-b]pyran] sind.

7. Verwendung der photochromen 2H-Naphtho[1,2-b]pyrane nach einem der Ansprüche 1 bis 6 in Kunststoffmaterialien.

8. Verwendung nach Anspruch 7, wobei das Kunststoffmaterial eine ophthalmische Linse ist.

## Claims

1. Photochromic 2H-naphtho[1,2-b]pyrans with the general formula (I): 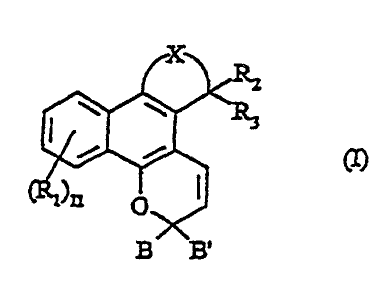 in which
X is a ring member with 2 to 4 saturated and/or unsaturated carbon atoms, of which a maximum of one carbon atom can be replaced by a heteroatom which is chosen from the group which comprises O, S and NR₄, R₄ representing an unbranched-chain or branched-chain (C₁-C₆)alkyl radical, a substituted or unsubstituted (C₅-C₇)cycloalkyl radical, a substituted or unsubstituted phenyl radical or a substituted or unsubstituted benzyl radical and the carbon atoms in the ring member X being able to be unsubstituted, mono- or di-substituted, the substituent or substituents then being able to be chosen from the group A which comprises (C₁-C₆)alkyl, (C₁-C₆)alkoxy, phenyl, bromine, chlorine and fluorine;
R₁ is a substituent which is chosen from the group A which comprises a (C₁-C₆)alkyl radical, a (C₁-C₆)alkoxy radical, a (C₃-C₇)cycloalkyl radical, which can have one or more heteroatoms, a phenyl radical, a hydroxy group, bromine, chlorine and fluorine, n being 0, 1 or 2;
R₂, R₃ are radicals which are independent of each other and which are chosen from the group G which comprises hydrogen, hydroxy, a (C₁-C₆)alkyl radical, a (C₁-C₆)alkoxy radical, a (C₃-C₇)cycloalkyl radical, an un-, mono- and di-substituted phenyl radical and an un-, mono- and di-substituted naphthyl radical and the aromatic radicals which are chosen from the group C which comprises benzene, naphthalene, phenanthrene, pyridine, quinoline, furane, thiophene, pyrrole, benzofurane, benzothiophene, indole and carbazole, the substituent or substituents of the aforementioned aromatic radicals being chosen from the group A;
or R₂ and R₃ form together, with inclusion of the spirocarbon atom, a 5 to 11-member ring, on which one or more aromatic or heteroaromatic ring systems can be anellated, the aromatic or heteroaromatic ringsystem(s) being one from the aforementioned group C;
or R₂ and R₃, by forming together a carbonyl group, stand for an oxygen atom;
with the proviso that if X is -(CH₂)₂- or -(CH₂)₃-, R₂ and R₃ are not simultaneously hydrogen;
B, B' are independently of each other chosen from the following groups a), b) or c),
a) being mono-, di- and tri-substituted aryl radicals, the aryl radical being phenyl or naphthyl;
b) being unsubstituted, mono- and di-substituted heteroaryl radicals, the heteroaryl radical being pyridyl, furanyl, benzofurane-2-yl, benzofurane-3-yl, thienyl, benzothien-2-yl or benzothien-3-yl; the substituents of the aryl or heteroaryl radicals in a) and b), which are chosen from a group which comprises hydroxy, amino, mono-(C₁-C₆)alkylamino, di-(C₁-C₆)alkylamino, being those on the aromatic un-, mono or di-substituted mono- and diphenylamino, piperidinyl, morpholinyl, carbazolyl, un-, mono- and di-substituted pyrryl, (C₁-C₆)alkyl, (C₁-C₆)alkoxy, bromine, chlorine and fluorine, the aforementioned aromatic and heteroaromatic ring systems being able to be substituted with (C₁-C₆)alkyl, (C₁-C₆)alkoxy, bromine, chlorine and fluorine;
c) Structural units with the following formulae (V) and (W) are 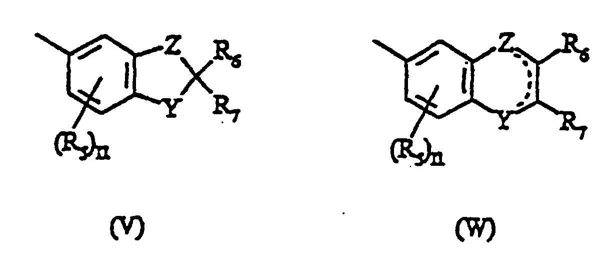 in which
Y and Z independently of each other are O, S, CH, CH₂ or NR₈, the radical R₈ is chosen from the group D which comprises (C₁-C₆)alkyl, (C₁-C₆)acyl and hydrogen, and R₅ is respectively a substituent from the group A, n being defined as above; and R₆ and R₇ independently of each other represent hydrogen and/or a (C₁-C₆)alkyl radical, with the proviso that if Y is NR₈ in the formula (V), Z is carbon;
or
d) B and B' together form an un-, mono- or di- substituted fluorene-9-ylide radical or a saturated hydrocarbon radical, which is C₃-C₁₂ spiromonocyclic, C₇-C₁₂ spiro-bicylic and/or C₇-C₁₂ spiro-tricyclic, the fluorene substituents being chosen from the group A.

2. Photochromic 2H-naphtho[1,2-b]pyrans according to claim 1, the carbon atoms in the ring member X being saturated.

3. Photochromic 2H-naphtho[1,2-b]pyrans according to claim 1 or 2, the carbon atoms in the ring member X being mono- or di-substituted, the substituent or substituents being chosen from the group A which comprises (C₁-C₆)alkyl, (C₁-C₆)alkoxy, phenyl, bromine, chlorine and fluorine.

4. Photochromic 2H-naphtho[1,2-b]pyrans according to one of the preceding claims, which have the general formula (II): 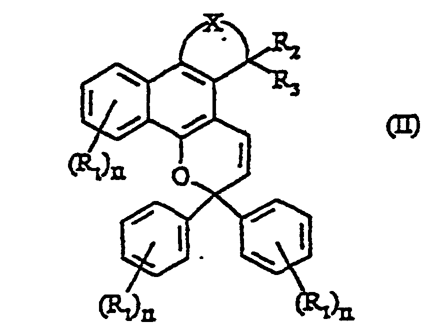 in which n, R₁, R₂, R₃ and X are defined as mentioned above, with the proviso that the radicals R₁ and n respectively can be the same or different.

5. Photochromic 2H-naphtho[1,2-b]pyrans according to one of the preceding claims, which have the general formula (III): 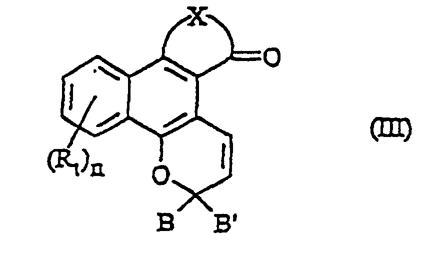 in which n, R₁, X, B and B' are defined as mentioned above.

6. Photochromic 2H-naphtho[1,2-b]pyrans, according to one of the preceding claims, which are
spiro-9'-xanthene-5-[2-(4-methoxyphenyl)-2-phenyl-5,6,7,8-tetrahydro-6,6,7,7-tetramethyl-phenanthro[9,10-b]pyran],
spiro-9'-fluorene-5-{2-[4-(morpholinyl)phenyl]-2-phenyl-5,6,7,8-tetrahydro-phenanthro[9,10-b]pyran},
5-hydroxy-2,2,5-triphenyl-5,6,7,8-tetrahydro-phenanthro[9,10-b]pyran,
spiro-9'-{(9,10-dihydroanthracene)-5-[2,2-bis(4-methoxyphenyl)}-cyclopenta[f]naphtho[1,2-b]pyran},
2-[4-(N-morpholinyl)phenyl]-2-phenyl-5-oxo-cyclopenta[f]naphthopyran,
2-[4-(N-morpholinyl)phenyl]-2-phenyl-5,6,7,8-tetrahydro-5-oxo-phenanthro[9,10-b]pyran,
spiro-9'-fluorene-5-[2-(4-methoxyphenyl)-2-phenyl-cyclopenta-[f]naphtho[1,2-b]pyran],
spiro-9'-xanthene-5-{2-[4-(N-morpholinyl)phenyl]-2-phenyl-5,6,7,8-tetrahydro-6,6,7,7-tetramethyl-phenanthro[9,10-b]pyran},
spiro-9'-fluorene-5-[2-(4-methoxyphenyl)-2-phenylcyclohepta[f]naphtho[1,2-b]pyran] and
spiro-9 '-fluorene-5-[2-(4-methoxyphenyl)-2-phenyl-oxepano[3,2-f]naphtho[1,2-b]pyran].

7. Use of the photochromic 2H-naphtho[1,2-b]pyrans according to one of the claims 1 to 6 in plastic materials.

8. Use according to claim 7, the plastic material being an ophthalmic lens.

## Revendications

1. 2H-naphte[1,2-b]pyranes photochromes présentant la formule générale I : 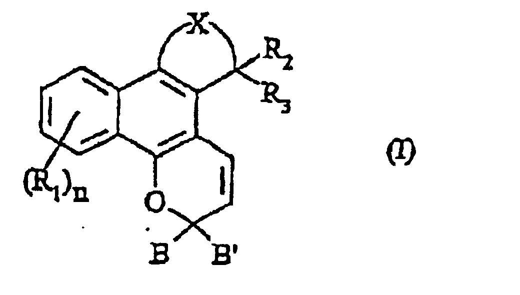 dans laquelle
X est un élément cyclique comprenant 2 à 4 atomes de carbone saturés et/ou insaturés, dont au maximum un atome peut être remplacé par un hétéroatome choisi parmi le groupe constitué de 0, S et NR₄, R₄ étant un radical alkyle en C₁ à C₆ linéaire ou ramifié, un radical cycloalkyle en C₅ à C₇ substitué ou non substitué, un radical phényle substitué ou non substitué ou un radical benzyle substitué ou non substitué, les atomes de carbone dans l'élément cyclique X pouvant être non substitués, monosubstitués ou disubstitués, le ou les substituants étant alors choisis parmi le groupe A, constitué de groupes alkyle en C₁ à C₆, alcoxy en C₁ à C₆, de phényle, de brome, de chlore et de fluor ;
R₁ est un substituant choisi parmi le groupe A, constitué d'un radical alkyle en C₁ à C₆, d'un radical alcoxy en C₁ à C₆, d'un radical cycloalkyle en C₃ à C₇, qui peut présenter un ou plusieurs hétéroatomes, d'un radical phényle, d'un groupe hydroxy, de brome, de chlore et de fluor, n étant égal à 0, 1 ou 2 ;
R₂ et R₃ sont, indépendamment l'un de l'autre, des radicaux choisis parmi le groupe G, constitué d'hydrogène, d'un groupe hydroxy, d'un radical alkyle en C₁ à C₆, d'un radical alcoxy en C₁ à C₆, d'un radical cycloalkyle en C₃ à C₇, d'un radical phényle non substitué, monosubstitué et disubstitué et d'un radical naphtyle non substitué, monosubstitué et disubstitué et des radicaux aromatiques, choisis parmi le groupe C, constitué de benzène, de naphtalène, de phénanthrène, de pyridine, de quinoléine, de furane, de thiophène, de pyrrole, de benzofurane, de benzothiophène, d'indol et de carbazol, le ou les substituants des radicaux aromatiques susmentionnés étant choisis parmi le groupe A ;
ou R₂ et R₃ sont ensemble, en incluant l'atome de carbone spiro, un cycle de 5 à 11 membres, sur lequel un ou plusieurs systèmes cycliques aromatiques ou hétéroaromatiques peuvent être cyclisés, le ou les systèmes cycliques aromatiques ou hétéroaromatiques étant l'un du groupe C susmentionné ;
ou R₂ et R₃ représentent, en formant ensemble un groupe carbonyle, un atome d'oxygène
à condition que, lorsque X est un groupe -(CH₂)₂- ou -(CH₂)₃-, R₂ et R₃ ne soient pas simultanément de l'hydrogène ;
B, B' sont, indépendamment l'un de l'autre, choisis parmi l'un des groupes a), b) ou c) suivants,
a) étant des radicaux aryle monosubstitués, disubstitués et trisubstitués, le radical aryle étant un radical phényle ou naphtyle,
b) étant des radicaux hétéroaryle non substitués, monosubstitués et disubstitués, le radical hétéroaryle étant un radical pyridyle, furannyle, benzofurane-2-yle, benzofurane-3-yle, thiényle, benzothiéne-2-yle ou benzothiéne-3-yle, les substituants des radicaux aryle ou hétéroaryle dans a) et b) étant ceux, choisis parmi un groupe constitué des groupes hydroxy, amino, mono(alkyle en C₁ à C₆)amino, di(alkyle en C₁ à C₆)amino, monophénylamino et diphénylamino non substitués, monosubstitués ou disubstitués sur le cycle aromatique, pipéridinyle, morpholinyle, carbazolyle, pyrryle non substitué, monosubstitué ou disubstitué, alkyle en C₁ à C₆, alcoxy en C₁ à C₆, de brome, de chlore et de fluor, les systèmes cycliques aromatiques et hétéroaromatiques susmentionnés pouvant être substitués par un groupe alkyle en C₁ à C₆, alcoxy en C₁ à C₆, du brome, du chlore et du fluor ;
c) des unités structurelles présentant les formules (V) et (W) suivantes 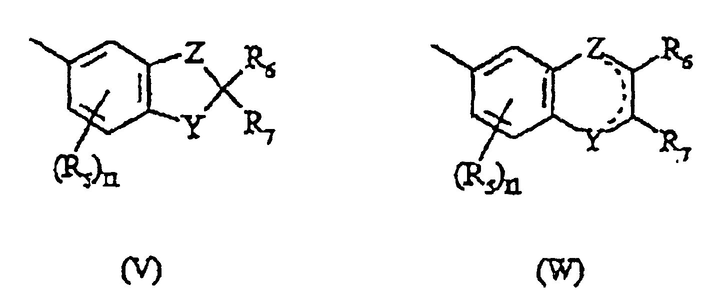 dans lesquelles
Y et Z représentent, indépendamment l'un de l'autre, O, S, CH, CH₂ ou NR₈, le radical R₈ est choisi parmi le groupe D, constitué des groupes alkyle en C₁ à C₆, acyle en C₁ à C₆ et d'hydrogène, R₅ est à chaque fois un substituant du groupe A, n étant tel que défini ci-dessus ; et R₆ et R₇ représentent, indépendamment l'un de l'autre, de l'hydrogène et/ou un radical alkyle en C₁ à C₆, à condition que, lorsque Y dans la formule (V) est un groupe NR₈, Z soit du carbone
ou
d) B et B' forment ensemble un radical fluoréne-9-ylidène non substitué, monosubstitué ou disubstitué, ou un radical hydrocarboné saturé, qui est spiro-monocyclique en C₃ à C₁₂, spiro-dicyclique en C₇ à C₁₂ et/ou spiro-tricyclique en C₇ à C₁₂, les substituants fluorène étant choisis parmi le groupe A.

2. 2H-naphto[1,2-b]pyranes photochromes selon la revendication 1, **caractérisés en ce que** les atomes de carbone dans l'élément cyclique X sont saturés.

3. 2H-naphto[1,2-b]pyranes photochromes selon la revendication 1 ou 2, les atomes de carbone dans l'élément cyclique X étant monosubstitués ou disubstitués, le ou les substituants étant choisis parmi le groupe A, constitué de groupes alkyle en C₁ à C₆, alcoxy en C₁ à C₆, phényle, de brome, de chlore et de fluor.

4. 2H-naphto[1,2-b]pyranes photochromes selon l'une quelconque des revendications précédentes, qui présentent la formule générale (II) : 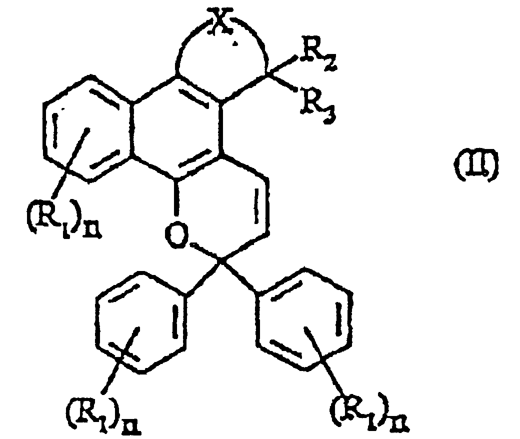 dans laquelle n, R₁, R₂, R₃ et X sont définis comme susmentionné, à condition que les radicaux R₁ et n puissent à chaque fois être identiques ou différents.

5. 2H-naphto[1,2-b]pyranes photochromes selon l'une quelconque des revendications précédentes, qui présentent la formule générale (III) : 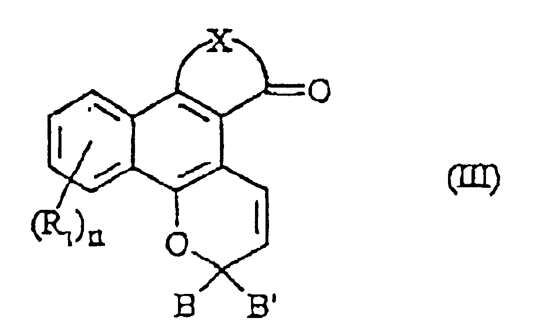 dans laquelle n, R₁, X, B et B' sont définis tels que susmentionné.

6. 2H-naphto[1,2-b]pyranes photochromes selon l'une quelconque des revendications précédentes, qui sont
du spiro-9'-xanthène-5-[2-(4-méthoxyphényl)-2-phényl-5,6,7,8-tétrahydro-6,6,7,7-tétraméthyl-phénanthro[9, 10-b]pyrane],
du spiro-9'-fluorène-5-{2-[4-(N-morpholinyl)phényl]-2-phényl-5,6,7,8-tétrahydro-phénanthro[9,10-b]pyrane},
du 5-hydroxy-2,2,5-triphényl-5,6,7,8-tétrahydro-phénanthro[9,10-b]pyrane,
du spiro-9'-{(9,10-dihydroanthracène)-5-[2,2-bis(4-méthoxyphényl)]-cyclopenta(f)naphto[1,2-b]pyrane},
du 2-[4-(N-morpholinyl)phényl]-2-phényl-5-oxo-cyclopenta[f]-naphtopyrane,
du 2-[4-(N-morpholinyl)phényl]-2-phényl-5,6,7,8-tétrahydro-5-oxo-phénanthro[9,10-b]pyrane,
du spiro-9'-fluorène-5-[2-(4-méthoxyphényl)-2-phénylcyclopenta-[f]naphto[1,2-b]pyrane],
du spiro-9'-xanthène-5-{2-[4-(N-morpholinyl)phényl]-2-phényl-5,6,7,8-tétrahydro-6,6,7,7-tétraméthylphénanthro[9,10-b]pyrane},
du spiro-9'-fluorène-5-[2-(4-méthoxyphényl)-2-phénylcyclohepta[f]naphto[1,2-b]pyrane] et
du spiro-9'-fluorène-5-[2-(4-méthoxyphényl)-2-phényloxepano-[3,2-f]naphto[1,2-b]pyrane].

7. Utilisation des 2H-naphto[1,2-b]pyranes photochromes selon l'une quelconque des revendications 1 à 6 dans des matériaux synthétiques.

8. Utilisation selon la revendication 7, le matériau synthétique étant une lentille ophtalmologique.
